# EUROPEAN PATENT SPECIFICATION

(11) **EP 2 857 016 B1**
(45) Date of publication and mention of the grant of the patent: **22.02.2017**
(21) Application number: 13784325.6
(22) Date of filing: 29.04.2013
(51) Int. Cl.: A61K 31/192, A61K 31/137, A61K 9/08

(54) **INJECTABLE PHARMACEUTICAL COMPOSITION OF DEXKETOPROFEN AND TRAMADOL**
INJIZIERBARE PHARMAZEUTISCHE ZUSAMMENSETZUNG AUS DEXKETOPROFEN UND TRAMADOL
COMPOSITION PHARMACEUTIQUE INJECTABLE DE DEXCÉTOPROFÈNE ET DE TRAMADOL

(30) Priority: 30.04.2012 ES 201200450
(43) Date of publication of application: 08.04.2015
(73) Proprietor: Farmalider, S.A., 28108 Alcobendas (Madrid) (ES)
(72) Inventor: SANZ MENÉNDEZ, Nuria, 28108 Alcobendas (Madrid) (ES); MUÑOZ RUÍZ, Ángel, 28108 Alcobendas (Madrid) (ES); MARTINEZ ALZAMORA, Fernando, 28108 Alcobendas (Madrid) (ES); GÓMEZ CALVO, Antonia, 28108 Alcobendas (Madrid) (ES); RIZO MARTÍNEZ, José Miguel, 28108 Alcobendas (Madrid) (ES)
(74) Representative: Rodriguez Oca, Jesus
(86) International application number: PCT/ES2013/000106
(87) International publication number: WO 2013/164498

(56) References cited:
- EP-A1- 0 546 676
- WO-A1-2008/150324
- US-A1- 2004 116 528
- TUNCER, S. ET AL.: 'Adding ketoprofen to intravenous patient-controlled analgesia with tramadol after major gynecological cancer surgery: A double-blinded, randomized, placebo-controlled clinical trial' EUROPEAN JOURNAL OF GYNAECOLOGICAL ONCOLOGY vol. 24, no. 2, 2003, pages 181 - 184, XP008142044
- ZEGPI, C. ET AL.: 'The effect of opioid antagonists on synergism between dexketoprofen and tramadol' PHARMACOLOGICAL RESEARCH vol. 60, no. 4, 2009, pages 291 - 295, XP026524587
- MIRANDA, H.F. ET AL.: 'Effects of tramadol and dexketoprofen on analgesia and gastrointestinal transit in mice' FUNDAMENTAL AND CLINICAL PHARMACOLOGY vol. 23, no. 1, 2009, pages 81 - 88, XP002699340

## Description

### Field of the invention

This invention refers to an injectable pharmaceutical composition containing, as active ingredient, a combination of dexketoprofen and tramadol, and to its use in pain treatment.

### Background of the invention

Tramadol is a pharmaceutical with analgesic properties that acts at the central level and is used in the treatment of moderate to severe pain of chronic or acute origin. It is also used in diagnostic exploration procedures that involve pain. Tramadol was originally described in a North American patent US3652589 filed by the company Grünenthal.

The mechanism of action of tramadol is often described as that of an atypical opioid, because it is neither opioid nor fully non-opioid. The mechanism of action of tramadol is dual; on the one hand it acts on µ-opioid receptors to which it binds with low affinity; and, on the other hand, it inhibits noradrenaline and serotonin reuptake, which increases the concentrations of these neurotransmitters en areas of the brain to reduce the pain threshold, as described for example in the article, Raffa et al, Opioid and nonopioid components independently contribute to the mechanism of action of tramadol, an 'atypical' opioid analgesic, J. Pharmacol. Exp. Ther., 1992, 260 (1), 275-85. Tramadol also has an antitussive effect.

It is well known that typical opioids such as morphine, codeine, oxycodone and hydrocodone, for example, produce characteristic undesirable side effects such as nausea and/or vomiting, sleepiness, respiratory depression, constipation, confusion and sedation. Long term use leads to physical dependency and addiction. Tramadol has less pronounced side effects and less frequent that those of typical opioids. Some cases of tramadol abuse have been described in opioid-dependent patients such as described in the article, Goeringer et al., Identification of tramadol and its metabolites in blood from drug-related deaths and drug-impaired drivers, J. Anal. Toxicol., 1997, 21 (7), 529-37. The more common adverse effects of tramadol are dizziness and/or vertigo, nausea and/or vomiting, constipation and headaches.

Dexketoprofen is a pharmaceutical belonging to the group of non-steroidal anti-inflammatory drugs (commonly called NSAIDs), with a strong anti-inflammatory, analgesic and antipyretic action. In particular, dexketoprofen belongs to the group of arylpropionic derivatives.

Dexketoprofen corresponds to the (S) enantiomeric form of ketoprofen, the latter being made up of a racemic mixture of the two enantiomeric (R) and (S) forms. Dexketoprofen is the active form of ketoprofen where, in common with the majority of arylpropionic derivatives, the therapeutic activity mainly lies in the enantiomeric (S) form.

Like the majority of NSAIDs, dexketoprofen exercises its analgesic action through inhibition of the cyclooxygenase enzyme (COX), responsible for prostaglandin synthesis. There are several variants of this enzyme, mainly cyclooxygenase-1 (COX-1), which is a constitutive enzyme that protects gastric mucosa in normal conditions and cyclooxygenase-2 (COX-2), which is an inducible enzyme mainly involved in inflammation. Dexketoprofen inhibits both COX-2 and COX-1, so that the analgesic, antipyretic and anti-inflammatory actions of dexketoprofen are attributed to inhibition of COX-2.

As with other NSAIDs, the main adverse effects attributed to dexketoprofen are localised at the level of the gastrointestinal tract and are derived from inhibition of COX-1.

In general, a strategy for minimising side effects of analgesic pharmaceuticals is to combine active ingredients with different mechanisms of action, for example opioid and non-opioid analgesics. It has been found that some of these combinations of active ingredients, in certain proportions, have a certain synergistic effect that enables achieving a desired analgesic action using a smaller amount of active ingredients, thereby reducing the unwanted adverse effects.

Thus for example, the article, Tuncer et al., Dexketoprofen for postoperative pain relief, Agri, 2006, 18 (3), 30-35, describes that the oral administration of dexketoprofen in patients with post-operative pain enabled reducing the consumption of tramadol, administered intravenously by an administration regime that was self-controlled by the patient (Patient Controlled Analgesia).

Also, in experimental pain models in mice, it has been found that there is a synergistic effect between dexketoprofen and tramadol when both active ingredients are combined in a weight ration of 1:1, as described in the article by Miranda et al., Effects of tramadol and dexketoprofen on analgesia and gastrointestinal transit in mice, Fundam. Clin. Pharmacol. 2009, 23, 81-88.

The article by Zegpi et al., The effect of opioid antagonists on synergism between dexketoprofen and tramadol; Pharmacological Research, 2009, 60, 4, 291-295, discloses an experimental study in mice using the formalin-induced orofacial pain model to assess the interaction between dexketoprofen co-administered with tramadol.

In certain circumstances, administration of drugs by the parenteral route is especially useful, particularly when oral administration is not possible or not advisable. Parenteral administrations includes, generally any injectable route: intradermal, subcutaneous, intramuscular and intravenous, the most advantageous being intravenous and intramuscular for analgesic, anti-inflammatory and/or antipyretic therapies.

The intravenous route enables rapid start of therapeutic action, given that all the administered drug reaches the systemic circulation immediately, without passing through a process of adsorption, further achieving better control of the concentration of the administered drug. This results in greater efficacy in pain control, particularly in the hospital setting.

Intramuscular administration also provides rapid and safe absorption of the drug.

Therefore, it is desirable to have parmaceuticals with the combination of dexketoprofen and tramadol ready for parenteral administration that enable a more effective and safe therapeutic action.

However, the combination of both active ingredients, especially in solution and in relatively high concentrations, is not trivial. Incompatibilities between the drugs have been described in the preparation of combined forms. Thus for example, patent application WO-A-2008/092219 describes preparing formulations of the combination of ketoprofen/tramadol for oral administration where the active ingredients are kept physically separated, because when they come into contact they interact, forming a thick viscous mass that is difficult to dissolve.

In hospital practice, dexketoprofen and tramadol are occasionally mixed in infusion bags, but this mixture is always prepared *in situ*, immediately or a short time before administration, with both active ingredients being at considerable dilution in the perfusion liquid, in order to minimise the possible problems of their interaction.

Thus there is the need to have available an injectable analgesic composition of a combination of dexketoprofen and tramadol with a suitable concentration of these active ingredients that facilitates their use for direct injection or with prior dilution in perfusion solution, that is stable over the long term, that is effective in pain control of moderate to severe intensity and that presents fewer side effects.

### Object of the invention

The object of this invention is an injectable pharmaceutical composition comprising:
a) dexketoprofen in the form of salt with tromethamine;
b) tramadol hydrochloride;
c) ethanol; and
d) water for injection;
characterised in that:
the concentration of dexketoprofen in the form of salt with tromethamine, expressed as the equivalent concentration of dexketoprofen, is between 15 mg and 75 mg per ml of solution; the concentration of tramadol hydrochloride is between 10 mg and 150 mg per ml of solution; and the concentration of ethanol is between 5 mg and 200 mg per ml of solution.

Also part of the object of the invention is a unit dosage pharmaceutical form containing this composition.

Also part of the object of this invention is a process for the preparation of this composition.

Also part of the object of the invention is the use of this composition for the preparation of a medicine for pain treatment of moderate to severe intensity, inflammation and/or fever.

### Detailed description of the invention

The object of this invention is an injectable pharmaceutical composition comprising: a) dexketoprofen in the form of salt with tromethamine; b) tramadol hydrochloride; c) ethanol; and d) water for injection; characterised in that: the concentration of dexketoprofen in the form of salt with tromethamine, expressed as the equivalent concentration of dexketoprofen, is between 15 mg and 75 mg per ml of solution; the concentration of tramadol hydrochloride is between 10 mg and 150 mg per ml of solution; and the concentration of ethanol is between 5 mg and 200 mg per ml of solution.

The authors of this invention have developed a pharmaceutical composition for administration by injection comprising a combination of dexketoprofen and tramadol dissolved in a mixture of water and ethanol that is stable even at relatively high concentrations of active ingredients, and enables achieving a rapid and effective therapeutic action with lower risk of side effects.

### Dexketoprofen

Dexketoprofen, or (S)2-(3-benzoylphenyl)-propionic acid, is the (S) enantiomer of ketoprofen.

Ketoprofen, or 2-(3-benzoylphenyl)-propionic acid is a compound that contains an asymmetric carbon or chiral centre, so that it normally occurs in a racemic mixture, that is, a mixture of 50% of its (S) and (R) enantiomers.

The preparation of ketoprofen is described in French patent application FR-A-1546478 and the separation of the two enantiomers is described in article Rendic et al., Chimia, 1975, 29, 170-72.

Dexketoprofen can also be prepared by enantiomer-selective synthesis as described in the article Comisso et al., Gazz. Chim. Ital., 1980, 110, 123.

Dexketoprofen is used in the form of its salt with tromethamine, also named trometamol, and that is commonly called dexketoprofen trometamol.

The concentration of dexketoprofen in the form of salt with tromethamine in the pharmaceutical composition of the invention is between 15 mg and 75 mg per ml of solution, expressed as the equivalent concentration of dexketoprofen; preferably between 18 mg and 40 mg per ml solution; more preferably between 22 mg and 30 mg, and in particular the concentration is 25 mg per ml solution.

### Tramadol

Tramadol is the International Nonproprietary Name (INN) normally used for the compound (±)-cis-2-[(dimethylamino)methyl]-1-(3-methoxyphenyl)-cyclohexanol.

Tramadol is commercially available and can also be prepared, for example according to the process described in the North American patent US3652589.

In the context of this invention, the term tramadol refers broadly to any of its solvates, polymorphs, stereoisomers, stereoisomeric mixtures and racemic forms.

Tramadol is in the form of its hydrochloride salt. With special preference, it is in the form of tramadol hydrochloride in racemic form.

The concentration of tramadol hydrochloride present in the composition of the invention is preferably between 10 mg and 150 mg per ml of solution, preferably between 15 mg and 35 mg per ml of solution, and more preferably between 20 mg and 30 mg per ml of solution; and in particular the concentration is 25 mg per ml of solution.

In another preferred embodiment of the invention, the concentration of tramadol, or one of its pharmaceutically acceptable salts, is between 40 mg and 60 mg per ml of solution, more preferably between 45 mg and 55 mg per ml of solution, and in particular the concentration is 50 mg per ml of solution.

### Cosolvent

The injectable composition of the invention comprises a mixture of water for injection and at least one cosolvent.

Water for injection is commercially available and, as is well known by an expert in the field, can be obtained by, for example, distillation or reverse osmosis.

The cosolvent is ethanol.

The cosolvent is used at a concentration of between 5 mg and 200 mg per ml of solution, particularly between 10 mg and 150 mg per ml of solution, and more particularly between 30 mg and 125 mg per ml solution.

### Injectable composition

The composition of the invention can additionally contain other components, for example isotonising agents, preservatives, buffers and/or pharmaceutically acceptable acids or bases to regulate the pH of the solutions.

In general, the composition of the invention has an osmolarity of between 200 and 380 mOsm/kg. If necessary, the composition of the invention may contain an isotonising agent to regulate the osmolarity of the solution to a value in this interval.

Suitable isotonising agents to be used in this invention can be selected from the group including sodium chloride, potassium chloride, ethanol, polyethylene glycol, propylene glycerol, sorbitol, inositol, mannitol, glycerol, glucose, fructose, dextrose, mannose, saccharose, lactose, maltose and mixtures of these.

In a preferred embodiment of the invention, the composition comprises an isotonising agent selected from the group consisting of sodium chloride, potassium chloride, glucose, dextrose and mixtures of these.

However, it is usually not necessary to add an additional isotonising agent because the cosolvent used in the composition exercises the role of the isotonising agent, already putting the compositions obtained in the range of osmolarity mentioned above.

Preferably, the composition of the invention has a pH between 6 and 8, particularly between 6.5 and 7.5 and more particularly between 6.7 and 7.3.

Optionally, the composition of the invention comprises a buffer system or buffer to regulate its pH in the interval of pH required. For this, any pharmaceutically acceptable buffering system may be used that enables regulating the pH in the specified interval. For example, the buffers that can be used include phosphate buffer, acetate, citrate or mixtures of these.

In a preferred embodiment, the composition of the invention comprises a phosphate buffer. This buffer can be prepared, for example, by dissolving monopotassium phosphate and sodium hydroxide.

The final pH of the solution can be adjusted by the addition of an acid or a base, usually a dilute aqueous solution of hydrochloric acid, for example at 10% or a dilute aqueous solution of sodium hydroxide, for example at 4%.

Optionally, the composition of the invention comprises a preservation agent. The preservative can be selected, for example, from benzalkonium chloride, benzyl alcohol, chlorobutanol, chlorocresol, cresol, ethanol, phenol, phenylethanol, sulphites, thimerosal, parabens, propylene glycol, sodium benzoate, phenylmercuric borate or phenylmercuric nitrate.

In a preferred embodiment of the invention, the injectable composition comprises:
- a concentration of dexketoprofen in the form of salt with tromethamine, expressed as an equivalent concentration of dexketoprofen, of between 18 mg/ml and 40 mg/ml, particularly between 22 mg/ml and 30 mg/ml and in particular of 25 mg/ml;
- a concentration of tramadol hydrochloride of between 15 mg/ml and 35 mg/ml, particularly between 20 mg/ml and 30 mg/ml and in particular of 25 mg/ml; and
- a concentration of ethanol of between 10 mg and 150 mg per ml of solution.

More preferably, the composition also comprises phosphate buffer.

In another preferred embodiment of the invention, the injectable composition comprises:
- a concentration of dexketoprofen in the form of salt with tromethamine, expressed as an equivalent concentration of dexketoprofen, of between 18 mg/ml and 40 mg/ml, particularly between 22 mg/ml and 30 mg/ml and in particular of 25 mg/ml;
- a concentration of tramadol hydrochloride of between 40 mg/ml and 60 mg/ml, particularly between 45 mg/ml and 55 mg/ml and in particular of 50 mg/ml; and
- a concentration of ethanol of between 10 mg and 150 mg per ml of solution.

More preferably, the composition also comprises phosphate buffer.

Despite containing significant amounts of dexketoprofen and tramadol, the compositions of the invention have good stability over at least three months in accelerated stability conditions, that is at 40 °C and 75% relative humidity and at 50°C.

The content of active ingredients can be analysed by HPLC (high pressure/resolution liquid chromatography techniques), well known by an expert in the field, and adjusting operational conditions by routine assays in a laboratory for the analysis of pharmaceutical active ingredients.

For example, dexketoprofen trometamol can be determined by the method described in the article Mulla et al., Development and Validation of HPLC method for simultaneous quantitation of paracetamol and dexketoprofen trometamol in bulk drug and formulation, Pharmacie Globale: (IJCP), Volume 2011, 7(09), 1-4. Tramadol can be analysed for example by the method described in Zaghloul et al., High performance liquid chromatographic determination of tramadol in pharmaceutical dosage forms, J. Liq. Chrom. Rel. Technol., 1997, 20, 779-87.

### Unit dosage pharmaceutical form

Also part of the object of the invention is a unit dosage pharmaceutical form that contains the composition described above.

This unit dosage pharmaceutical form comprises a volume of the composition suitable for administering the desired dose of the active ingredients. Preferably, the composition is dosed in vials or preloaded syringes.

In a preferred embodiment, the volume of the unit dosage pharmaceutical form is between 1 and 5 ml.

Preferably, the unit dosage pharmaceutical form comprises an amount of dexketoprofen or of one of its pharmaceutically acceptable salts of between 15 mg and 100 mg, particularly between 40 mg and 60 mg, and more particularly of 50 mg, expressed as dexketoprofen equivalent amount; and an amount of tramadol, or one of its pharmaceutically acceptable salts, of between 20 mg and 150 mg, particularly between 30 mg and 110 mg, expressed as an equivalent amount of tramadol hydrochloride, preferably in a volume of between 1 and 5 ml.

In a preferred embodiment, the injectable composition of the invention is dispensed in a unit dosage pharmaceutical form that comprises an amount of tramadol, expressed as an equivalent weight of tramadol hydrochloride of between 40 mg and 60 mg, and more particularly of 50 mg.

In another preferred embodiment, the injectable composition of the invention is dosed in a unit dosage pharmaceutical form that comprises an amount of tramadol, expressed as an equivalent weight of tramadol hydrochloride of between 90 mg and 110 mg, and more particularly of 100 mg.

Preferably, the unit dosage pharmaceutical form comprises a dose of 50 mg of dexketoprofen, or one of its pharmaceutically acceptable salts, expressed as an equivalent weight of dexketoprofen; and a dose of tramadol, or one of its pharmaceutically acceptable salts, expressed as an equivalent weight of tramadol hydrochloride, selected between 50 mg or 100 mg, in such a way as to have available the combination of 50 mg of dexketoprofen with 50 mg of tramadol and the combination of 50 mg of dexketoprofen with 100 mg of tramadol. Preferably, the dexketoprofen is in the form of dexketoprofen trometamol and the tramadol is in the form of tramadol hydrochloride.

In a preferred embodiment of the invention, the unit dosage pharmaceutical form comprises:
- an amount of dexketoprofen in the form of salt with tromethamine, expressed as an equivalent weight of dexketoprofen, of between 45 mg and 55 mg, preferably of 50 mg;
- an amount of tramadol hydrochloride
   of between 45 mg and 55 mg, preferably of 50 mg.

- an amount of ethanol of between 18 mg and 330 mg; and
- water for injection to a volume of between 1.8 ml and 2.2 ml.

More preferably, the composition also comprises phosphate buffer.

In another preferred embodiment of the invention, the unit dosage pharmaceutical form comprises:
- an amount of dexketoprofen in the form of salt with tromethamine, expressed as an equivalent weight of dexketoprofen, of between 45 mg and 55 mg, preferably of 50 mg;
- an amount of tramadol hydrochloride of between 95 mg and 105 mg, preferably of 100 mg;
- an amount of ethanol of between 18 mg and 330 mg; and
- water for injection to a volume of between 1.8 ml and 2.2 ml.

More preferably, the composition also comprises phosphate buffer.

### Preparation of the injectable composition

Also part of the object of the invention is a process for preparing a pharmaceutical composition of the invention.

The process for preparing the compositions of the invention comprise dissolving the dexketoprofen in the form of salt with tromethamine, and the tramadol hydrochloride, in a mixture containing water for injection and a cosolvent.

Optionally, the process comprises additionally adding a buffering agent to the mixture of water for injection and of cosolvent, adjusting the pH to a value of between 6 and 8.

A usual way of preparing the composition of the invention comprises having water for injection in a reactor with a stirrer and then adding the cosolvent. Optionally, adding a buffering agent and adjusting the pH value to the range 6-8 if necessary with a dilute solution of HCl or NaOH. Next, adding to the solution, dexketoprofen, or one of its pharmaceutically acceptable salts, stirring until complete solution, and the tramadol, or one of its pharmaceutically acceptable salts, maintaining stirring until complete solution.

Finally, if necessary, adding additional water for injection to obtain the desired concentration of the active ingredients.

The solution obtained can be sterilised, for example by filtration. Next, the resulting solution can be dispensed into unit dosage pharmaceutical forms, in a suitable container, for example in sterile vials or syringes.

Preferably, the composition is dosed at in an amount of 2 ml of solution in each dose, although it is possible to use a unit dosage with a higher volume, for example of up to 5 ml.

Optionally, the vials or syringes containing the composition of the invention are sterilised in an autoclave, for example by treatment at a temperature of approximately 121 °C for approximately 20 minutes.

### Use of the injectable composition

The compositions of the invention have an analgesic effect and are useful for pain treatment, particularly chronic or acute pain, of moderate to severe intensity, and also of inflammation and fever and, generally, of any disease susceptible to treatment with tramadol and dexketoprofen.

Therefore, also part of the object of the invention is the use of the composition of the invention in the treatment of pain, inflammation and/or fever or any disease susceptible to being treated with tramadol and/or dexketoprofen. Or, expressed alternatively, part of the object of the invention is the use of the composition of the invention for the preparation of a medicine for the treatment of pain, inflammation and/or fever or any disease susceptible of being treated with tramadol and/or dexketoprofen. Or alternatively, part of the object of the invention is a method for the treatment of pain, inflammation and/or fever or any disease susceptible of treatment with tramadol and/or dexketoprofen comprising the administration of a composition of the invention to a mammal, preferably a person, that needs it.

The compositions of the invention can be used for the treatment of different types of pain cause by different ailments such as headache, toothache, earache, migraine, muscle pain, joint pain, neuropathic pain, post-traumatic pain, post-partum pain, post-surgical pain, acute myocardial infarction pain and cancer pain. It can also be used as a preoperative analgesic, as a complement to surgical anaesthesia and in diagnostic exploratory procedures that involve pain. Furthermore, the compositions can be used as anti-inflammatories and, mainly because of the action of dexketoprofen, as antipyretics, because they are useful in the treatment of inflammation and/or fever caused by different ailments such as rheumatoid arthritis (including juvenile rheumatoid arthritis), osteoarthritis, ankylosing spondylitis, arthritis and other acute or chronic rheumatism conditions, bursitis, synovitis, capsulitis or other inflammatory lesions of traumatic or sports origin.

The compositions of the invention can also be useful in the treatment of other ailments that can be relieved with dexketoprofen or tramadol.

The term "treatment" includes both treatment of established symptoms and prophylactic treatment. Preferably, it is used in the treatment of established symptoms.

The pharmaceutical composition of this invention can be administered by the parenteral route. In the context of this invention, the terms parenteral or injectable are used indistinctly, referring to administration by any intramuscular, intravenous, subcutaneous or intradermal routes. Preferably, the composition of the invention is administered by the intramuscular route or by the intravenous route, either by direct intravenous injection or by intravenous infusion, with prior dilution in solutions suitable for perfusion well known by the expert in the field, for example by aqueous 0.9% sodium chloride solution, Lactated Ringer's solution or aqueous 5% glucose solution.

Various non-limiting examples are provided below to illustrate the invention.

### Examples

### Example 1: Injectable composition containing 25 mg/ml of dexketoprofen and 25 mg/ml of tramadol hydrochloride

An injectable solution was prepared using the components listed in the following table:

| **Ingredient** | **Amount (mg/ml)** |
|---|---|
| Dexketoprofen trometamol | 36.9 |
| Tramadol hydrochloride | 25.0 |
| Ethanol | 100.0 |
| Monopotassium phosphate | 1.7 |
| Sodium hydroxide | 0.3 |
| Water for injection | q.s. |

One part of water for injection was placed in a reactor, corresponding to approximately 60% of the total. Under continuous stirring, ethanol was added and then, monosodium phosphate and sodium hydroxide, until fully dissolved. The pH of the solution was checked and adjusted to 7.0 by the addition of sodium hydroxide. Next were added consecutively, dexketoprofen trometamol and tramadol hydrochloride, stirring until each addition was fully dissolved. The volume was made up with water for injection and the solution obtained sterilised by filtration through a 0.22 micron filter.

The resulting solution was dispensed into transparent glass vials in a volume of 2 ml solution per vial, each containing 50 mg dexketoprofen and 50 mg tramadol hydrochloride.

### Example 2: iniectable solution containing 25 mg/ml dexketoprofen and 50 mg/ml tramadol hydrochloride

An injectable solution was prepared using the components listed in the following table:

| **Ingredient** | **Amount (mg/ml)** |
|---|---|
| Dexketoprofen trometamol | 36.9 |
| Tramadol hydrochloride | 50 |
| Ethanol | 100 |
| Monopotassium phosphate | 1.7 |
| Sodium hydroxide | 0.3 |
| Water for injection | q.s. |

For the preparation of this composition, a process analogous to that described in Example 1 was followed. The resulting solution was dispensed into transparent glass vials at 2 ml solution in each vial, each containing 50 mg dexketoprofen and 100 mg tramadol hydrochloride.

### Example 3: injectable solution containing 25 mg/ml dexketoprofen and 25 mg/ml tramadol hydrochloride

An injectable solution was prepared using the components listed in the following table:

| **Ingredient** | **Amount (mg/ml)** |
|---|---|
| Dexketoprofen trometamol | 36.9 |
| Tramadol hydrochloride | 25.0 |
| Ethanol | 50 |
| Monopotassium phosphate | 1.7 |
| Sodium hydroxide | 0.3 |
| Water for injection | q.s. |

For the preparation of this composition, a process analogous to that described in Example 1 was followed. The resulting solution was dispensed into transparent glass vials at 2 ml solution per vial, each containing 50 mg dexketoprofen and 50 mg tramadol hydrochloride.

### Example 4: injectable solution containing 25 mg/ml dexketoprofen and 50 mg/ml tramadol hydrochloride

An injectable solution was prepared using the components listed in the following table:

| **Ingredient** | **Amount (mg/ml)** |
|---|---|
| Dexketoprofen trometamol | 36.9 |
| Tramadol hydrochloride | 50 |
| Ethanol | 50 |
| Monopotassium phosphate | 1.7 |
| Sodium hydroxide | 0.3 |
| Water for injection | q.s. |

For the preparation of this composition, a process analogous to that described in Example 1 was followed. The resulting solution was dispensed into transparent glass vials at 2 ml solution in each vial, each containing 50 mg dexketoprofen and 100 mg tramadol hydrochloride.

### Example 5: iniectable solution containing 25 mg/ml dexketoprofen and 25 mg/ml tramadol hydrochloride

An injectable solution was prepared using the components listed in the following table:

| **Ingredient** | **Amount (mg/ml)** |
|---|---|
| Dexketoprofen trometamol | 36.9 |
| Tramadol hydrochloride | 25 |
| Ethanol | 10 |
| Monopotassium phosphate | 1.7 |
| Sodium hydroxide | 0.3 |
| Water for injection | q.s. |

For the preparation of this composition, a process analogous to that described in Example 1 was followed. The resulting solution was dispensed into transparent glass vials at 2 ml solution in each vial, each containing 50 mg dexketoprofen and 50 mg tramadol hydrochloride.

### Example 6: stability of injectable compositions of dexketoprofen and tramadol hydrochloride

The vials with injectable compositions of dexketoprofen and tramadol hydrochloride prepared in the previous examples were subjected to accelerated stability assays for 3 months at a temperature of 40 °C under a relative humidity of 75% and also at 50 °C.

The content of each of the active ingredients was determined by HPLC.

The following table shows the content, in terms of the theoretical content of the active ingredients, of these stability assays:

### 1) Dexketoprofen, 40 °C and 75% relative humidity

| Composition | Start | 1 month | 3 months |
|---|---|---|---|
| Example 1 | 98.99 | 100.89 | 99.68 |
| Example 2 | 98.04 | 100.60 | 98.68 |
| Example 3 | 100.85 | 100.41 | 96.14 |
| Example 4 | 95.65 | 98.56 | 97.75 |
| Example 5 | 99.21 | 95.89 | 95.71 |

### 2) Tramadol. 40 °C and 75 % relative humidity

| Composition | Start | 1 month | 3 months |
|---|---|---|---|
| Example 1 | 98.28 | 102.16 | 102.88 |
| Example 2 | 99.05 | 100.91 | 101.40 |
| Example 3 | 99.95 | 101.70 | 98.99 |
| Example 4 | 97.15 | 99.36 | 100.33 |
| Example 5 | 97.51 | 96.00 | 98.20 |

### 3) Dexketoprofen, 50 °C

| Composition | Start | 1 month | 3 months |
|---|---|---|---|
| Example 1 | 98.99 | 100.51 | 99.32 |
| Example 2 | 98.04 | 100.99 | 98.91 |
| Example 3 | 100.85 | 97.60 | 100.09 |
| Example 4 | 95.65 | 98.69 | 97.43 |
| Example 5 | 99.21 | 93.92 | 95.50 |

### 4) Tramadol, 50 °C

| Composition | Start | 1 month | 3 months |
|---|---|---|---|
| Example 1 | 98.28 | 101.84 | 102.15 |
| Example 2 | 99.05 | 101.38 | 101.68 |
| Example 3 | 99.95 | 98.87 | 102.90 |
| Example 4 | 97.15 | 99.30 | 99.54 |
| Example 5 | 97.51 | 95.90 | 98.36 |

It can be observed that the compositions of the invention have good stability over the whole period evaluated, despite including significant amounts of dexketoprofen trometamol and tramadol hydrochloride.

## Claims

1. Injectable pharmaceutical composition comprising:
a) dexketoprofen in the form of salt with tromethamine;
b) tramadol hydrochloride;
c) ethanol; and
d) water for injection;
**characterised in that**:
the concentration of dexketoprofen in the form of salt with tromethamine, expressed as the equivalent concentration of dexketoprofen, is between 15 mg and 75 mg per ml of solution;
the concentration of tramadol hydrochloride is between 10 mg and 150 mg per ml of solution; and
the concentration of ethanol is between 5 mg and 200 mg per ml of solution.

2. Composition of claim 1, wherein the concentration of dexketoprofen in the form of salt with tromethamine, expressed as an equivalent concentration of dexketoprofen, is between 18 mg and 40 mg per ml of solution.

3. Composition of claim 2, wherein the concentration of dexketoprofen in the form of salt with tromethamine, expressed as an equivalent concentration of dexketoprofen, is between 22 mg and 30 mg per ml of solution.

4. Composition of any of claims 1 to 3, wherein the concentration of tramadol hydrochloride is between 15 mg and 35 mg per ml of solution.

5. Composition of claim 4, wherein the concentration of tramadol hydrochloride is between 20 mg and 30 mg per ml of solution.

6. Composition of any of the claims 1 to 3, wherein the concentration of tramadol hydrochloride is between 40 mg and 60 mg per ml of solution.

7. Composition of claim 6, wherein the concentration of tramadol hydrochloride is between 45 mg and 55 mg per ml of solution.

8. Composition of any of claims 1 to 7, wherein the ethanol is at a concentration of between 10 mg and 150 mg per ml of solution.

9. Composition of claim 1, wherein it comprises:
- a concentration of 25 mg/ml of dexketoprofen in the form of salt with tromethamine, expressed as an equivalent concentration of dexketoprofen;
- a concentration of 25 mg/ml of tramadol hydrochloride; and
- a concentration of ethanol of between 10 mg and 150 mg per ml of solution.

10. Composition of claim 1, wherein it comprises:
- a concentration of 25 mg/ml of dexketoprofen in the form of salt with tromethamine, expressed as an equivalent concentration of dexketoprofen;
- a concentration of 50 mg/ml of tramadol hydrochloride; and
- a concentration of ethanol of between 10 mg and 150 mg per ml of solution.

11. Composition of any of the claims 1 to 10, wherein it comprises a suitable buffering agent to regulate the pH in the interval of 6-8.

12. Composition of claim 11, wherein the buffer comprises phosphate buffer.

13. Unit dosage pharmaceutical form comprising a composition of claim 1, wherein it comprises an amount of dexketoprofen in the form of salt with tromethamine, expressed as an equivalent weight of dexketoprofen, of between 15 mg and 100 mg; and an amount of tramadol hydrochloride of between 20 and 150 mg.

14. Unit dosage pharmaceutical form of claim 13, wherein it comprises an amount of dexketoprofen in the form of salt with tromethamine, expressed as an equivalent weight of dexketoprofen, of between 40 mg and 60 mg; and an amount of tramadol hydrochloride of between 30 mg and 110 mg.

15. Unit dosage pharmaceutical form of any of the claims 13 or 14, wherein its volume is between 1 and 5 ml.

16. Unit dosage pharmaceutical form of claim 13, wherein it comprises:
- between 45 and 55 mg of dexketoprofen in the form of salt with tromethamine, expressed as an equivalent weight of dexketoprofen;
- between 45 mg and 55 mg of tramadol hydrochloride;
- between 18 mg and 330 mg of ethanol; and
- water for injection to a volume of between 1.8 ml and 2.2 ml.

17. Unit dosage pharmaceutical form of claim 13 wherein it comprises:
- between 45 and 55 mg of dexketoprofen in the form of salt with tromethamine, expressed as an equivalent weight of dexketoprofen;
- between 95 mg and 105 mg of tramadol hydrochloride;
- between 18 mg and 330 mg of ethanol; and
- water for injection to a volume of between 1.8 ml and 2.2 ml.

18. Unit dosage pharmaceutical form of any of the claims 16 or 17, wherein it comprises phosphate buffer.

19. Process for preparing a composition of any of the claims 1 to 12, wherein it comprises dissolving the dexketoprofen in the form of salt with tromethamine and tramadol hydrochloride in a mixture formed by water for injection and ethanol.

20. Composition of any of the claims 1 to 12 or unit dosage pharmaceutical form of any of the claims 13 to 18 for use in the treatment of pain, inflammation and fever, or any disease susceptible to treatment with tramadol and/or dexketoprofen.

## Patentansprüche

1. Spritzfähige pharmazeutische Zusammensetzung enthaltend:
a) Dexketoprofen in Form eines Salzes mit Tromethamin
b) Tramadol Hydrochlorid
c) Ethanol und
d) Wasser für die Injektion
**gekennzeichnet dadurch, dass**
die Konzentration von Dexketoprofen in Form eines Salzes mit Tromethamin, ausgedrückt als die äquivalente Konzentration von Dexketoprofen, zwischen 15 mg und 75 mg pro ml Lösung liegt,
dass die Konzentration von Tramadol Hydrochlorid zwischen 10 mg und 150 mg pro ml Lösung liegt, und
die Konzentration von Ethanol zwischen 5 mg und 200 mg pro ml Lösung liegt.

2. Zusammensetzung gemäss Anspruch 1, in der die Konzentration von Dexketoprofen in Form eines Salzes mit Tromethamin, ausgedrückt als eine äquivalente Konzentration von Dexkeotoprofen, zwischen 18 mg und 40 mg pro ml Lösung liegt.

3. Zusammensetzung gemäss Anspruch 2, in der die Konzentration von Dexketoprofen in Form eines Salzes mit Tromethamin, ausgedrückt als äquivalente Konzentration von Dexketoprofen, zwischen 22 mg und 30 mg pro ml Lösung liegt.

4. Zusammensetzung übereinstimmend mit irgendeinem der Ansprüche 1 bis 3, in der die Konzentration von Tramadol Hydrochlorid zwischen 15 mg und 35 mg pro ml Lösung liegt.

5. Zusammensetzung gemäss Anspruch 4, in der die Konzentration von Tramadol Hydrochlorid zwischen 20 mg und 30 mg pro ml Lösung liegt.

6. Zusammensetzung gemäss irgendeinem der Ansprüche 1 bis 3, in der die Konzentration von Tramadol Hydrochlorid zwischen 40 mg und 60 mg pro ml Lösung liegt.

7. Zusammensetzung gemäss Anspruch 6, in der die Konzentration von Tramadol Hydrochlorid zwischen 45 mg und 55 mg pro ml Lösung liegt.

8. Zusammensetzung gemäss irgendeinem der Ansprüche 1 bis 7, in der das Ethanol in einer Konzentration von 10 mg bis 150 mg pro ml Lösung vorliegt.

9. Zusammensetzung gemäss Anspruch 1, die folgendes enthält:
- eine Konzentration von 25 mg/ml Dexketoprofen in Form eines Salzes mit Tromethamin, ausgedrückt als eine äquivalente Konzentration von Dexketoprofen;
- eine Konzentration von 25 mg/ml Tramadol Hydrochlorid, und
- eine Ethanolkonzentration von 10 mg bis 150 mg pro ml Lösung.

10. Zusammensetzung gemäss Anspruch 1, die folgendes enthält:
- eine Konzentration von 25 mg/ml Dexketoprofen in Form eines Salzes mit Tromethamin, ausgedrückt als eine äquivalente Konzentration von Dexketoprofen;
- eine Konzentration von 50 mg/ml Tramadol Hydrochlorid, und
- eine Ethanolkonzentration von 10 mg bis 150 mg pro ml Lösung.

11. Zusammensetzung gemäss irgendeinem der Ansprüche 1 bis 10, die eine Puffersubstanz für die Einstellung des pH-Wertes zwischen 6-8 enthält.

12. Zusammensetzung gemäss Anspruch 11, wobei die Puffersubstanz einen Phosphatpuffer enthält.

13. Pharmazeutische Form einer Einheitsdosis, wobei diese eine Zusammensetzung gemäss Anspruch 1 enthält, bei der sie Dexketoprofen in Form eines Salzes mit Tromethamin, ausgedrückt als ein äquivalentes Gewicht von Dexketoprofen, in einer Menge von 15 mg bis 100 mg und Tramadol Hydrochlorid in einer Menge von 20 bis 150 mg enthält.

14. Pharmazeutische Form einer Einheitsdosis Anspruch 13, wobei diese Dexketoprofen in Form eines Salzes mit Tromethamin, ausgedrückt als ein äquivalentes Gewicht von Dexketoprofen, in einer Menge von 40 mg bis 60 mg und Tramadol Hydrochlrid in einer Menge von 30 mg bis 110 mg enthält.

15. Pharmazeutische Form einer Einheitsdosis gemäss irgendeinem der Ansprüche 13 oder 14, wobei ihr Volumen zwischen 1 und 5 ml beträgt.

16. Pharmazeutische Form einer Einheitsdosis gemäss Anspruch 13, wobei sie folgendes enthält:
- Dexketoprofen in Form eines Salzes mit Tromethamin, ausgedrückt als äquivalentes Gewicht von Dexketoprofen, in einer Menge von 45 bis 55 mg;
- Tramadol Hydrochlorid in einer Menge von 45 mg bis 55 mg,
- Ethanol in einer Menge von 18 mg bis 330 mg, und
- Wasser für die Injektion bis zu einem Volumen von 1,8 ml bis 2,2 ml.

17. Pharmazeutische Form einer Einheitsdosis übereinstimmend mit Anspruch 13, wobei dieselbe folgendes enthält:
- Dexketoprofen in Form eines Salzes mit Tromethamin, ausgedrückt als äquivalentes Gewicht von Dexketoprofen, in einer Menge von 45 mg bis 55 mg;
- Tramadol Hydrochlorid in einer Menge von 95 mg bis 105 mg;
- Ethanol in einer Menge von 18 mg bis 330 mg, und
- Wasser für die Injektion bis zu einem Volumen von 1,8 ml bis 2,2 ml.

18. Pharmazeutische Form einer Einheitsdosis gemäss Anspruch 16 oder 17, wobei dieselbe einen Phosphatpuffer enthält.

19. Verfahren für die Zubereitung einer Zusammensetzung gemäss irgendeiner der Ansprüche 1 bis 12, wobei dabei das Dexketoprofen in Form eines Salzes mit Tromethamin und das Tramadol Hydrochlorid in einer Mischung in Wasser für die Injektion und Ethanol aufgelöst wird.

20. Zusammensetzung gemäss irgendeiner der Ansprüche 1 bis 12 oder pharmazeutische Form einer Einheitsdosis gemäss irgendeiner der Ansprüche 13 bis 18 für die Behandlung von Schmerzen, Entzündungen oder Fieber oder jeglicher anderen Krankheit, die mit Tramadol und/oder Dexketoprofen behandelt werden kann.

## Revendications

1. Composition pharmaceutique injectable comprenant :
a) du dexkétoprofène sous forme de sel de trométhamine ;
b) du chlorhydrate de tramadol ;
c) de l'éthanol ; et
d) de l'eau pour injection;
**caractérisée en ce que** :
la concentration de dexkétoprofène sous forme de sel de trométhamine, exprimée comme la concentration équivalente de dexkétoprofène, est de 15 mg à 75 mg par mL de solution ;
la concentration de chlorhydrate de tramadol est de 10 mg à 150 mg par mL de solution ; et
la concentration d'éthanol est de 5 mg à 200 mg par mL de solution.

2. La composition de la revendication 1 où la concentration de dexkétoprofène sous forme de sel de trométhamine, exprimée en concentration équivalente de dexkétoprofène, est de 18 mg à 40 mg par mL de solution.

3. La composition de la revendication 2 où la concentration de dexkétoprofène sous forme de sel de trométhamine, exprimée en concentration équivalente de dexkétoprofène, est de 22 mg à 30 mg par mL de solution.

4. La composition des revendications 1 à 3 où la concentration de chlorhydrate de tramadol est de 15 mg à 35 mg par mL de solution.

5. La composition de la revendication 4 où la concentration de chlorhydrate de tramadol est de 20 mg à 30 mg par mL de solution.

6. La composition des revendications 1 à 3 où la concentration de chlorhydrate de tramadol est de 40 mg à 60 mg par mL de solution.

7. La composition de la revendication 6 où la concentration de chlorhydrate de tramadol est de 45 mg à 55 mg par mL de solution.

8. La composition des revendications 1 à 7 où la concentration d'éthanol est de 10 mg à 150 mg par mL de solution.

9. La composition de la revendication 1 qui comprend :
- une concentration de 25 mg/mL de dexkétoprofène sous forme de sel de trométhamine, exprimée en concentration équivalente de dexkétoprofène ;
- une concentration de 25 mg/mL de chlorhydrate de tramadol ; et
- une concentration d'éthanol de 10 mg à 150 mg par mL de solution.

10. La composition de la revendication 1 qui comprend :
- une concentration de 25 mg/mL de dexkétoprofène sous forme de sel de trométhamine, exprimée en concentration équivalente de dexkétoprofène ;
- une concentration de 50 mg/mL de chlorhydrate de tramadol ; et
- une concentration d'éthanol de 10 mg à 150 mg par mL de solution.

11. La composition des revendications 1 à 10 qui comprend un agent tampon approprié, destiné à régler le pH dans l'intervalle de 6-8.

12. La composition de la revendication 11 dans laquelle le tampon comprend tampon phosphate.

13. Forme pharmaceutique en dose unitaire comprenant une composition de la revendication 1, qui comprend une quantité de dexkétoprofène sous forme de sel avec de la trométhamine, exprimée comme un poids équivalent de dexkétoprofène, compris entre 15 mg et 100 mg ; et une quantité de chlorhydrate de tramadol comprise entre 20 mg et 150 mg.

14. La forme pharmaceutique en dose unitaire de la revendication 13 comprenant une quantité de dexkétoprofène sous forme de sel de trométhamine, exprimée comme un poids équivalent de dexkétoprofène, compris entre 40 mg et 60 mg ; et une quantité de chlorhydrate de tramadol comprise entre 30 mg et 110 mg.

15. La forme pharmaceutique en dose unitaire des revendications 13 ou 14 avec un volume de 1 à 5 mL.

16. La forme pharmaceutique en dose unitaire de la revendication 13 comprenant :
- de 45 mg à 55 mg de dexkétoprofène sous forme de sel de trométhamine, exprimé comme un poids équivalent de dexkétoprofène ;
- de 45 mg à 55 mg de chlorhydrate de tramadol ;
- de 18 mg à 330 mg d'éthanol ; et
- de l'eau pour injection à un volume compris entre 1,8 mL et 2,2 mL.

17. La forme pharmaceutique en dose unitaire de la revendication 13 comprenant :
- de 45 mg à 55 mg de dexkétoprofène sous forme de sel de trométhamine, exprimé comme un poids équivalent de dexkétoprofène ;
- de 95 mg à 105 mg de chlorhydrate de tramadol ;
- de 18 mg à 330 mg d'éthanol ; et
- de l'eau injection à un volume compris entre 1,8 mL et 2,2 mL.

18. La forme pharmaceutique en dose unitaire des revendications 16 ou 17 comprenant tampon phosphate.

19. Procédé d'élaboration d'une composition des revendications 1 à 12 comprenant la dissolution du dexkétoprofène sous forme de sel avec de la trométhamine et du chlorhydrate de tramadol dans un mélange à base d'eau pour injection et d'éthanol.

20. La composition de l'une quelconque des revendications 1 à 12 ou la forme pharmaceutique en dose unitaire de l'une quelconque des revendications 13 à 18 pour une utilisation dans le traitement de la douleur, des inflammations et de la fièvre, ou de toute maladie susceptible d'être traitée avec du tramadol et/ou du dexkétoprofène.
